# EUROPEAN PATENT APPLICATION

(11) **EP 4 461 204 A1**
(43) Date of publication of application: **13.11.2024**
(21) Application number: 22896894.7
(22) Date of filing: 17.11.2022
(51) Int. Cl.: A61B 1/12, A61B 1/253, A61C 9/00, A61C 17/022

(54) **COMPRESSED AIR BLOWER FOR ODONTOLOGICAL INTRAORAL SCANNER END-PIECE**

(30) Priority: 26.11.2021 BR 102021023828
(71) Applicant: Alfredo Risso, Alvaro, 03308050 São Paulo (BR)
(72) Inventor: KLAUS RISSO, Leon, 03308050 São Paulo (BR)
(74) Representative: Martin Santos, Victoria Sofia
(86) International application number: PCT/BR2022/050447
(87) International publication number: WO 2023/092206

(57) **Abstract**

A compressed air blower (10) formed by a pair of cannulas (11A) and (11B) coupled to the body of the tip (22) of the dental scanner (20) composing a region (RL) around the opening (22B) devoid of salivary flow (F1), blood (S1) or any other fluid, region (RL) for data collection and obtaining distortion-free (IG) three-dimensional/two-dimensional images of the patient's mouth region (PC) by means of air jets (AR) coming from openings (11a3) and (11b3) of the cannulas (11A) and (11B).

## Description

### TECHNICAL FIELD OF THE INVENTION

The present invention patent deals with a compressed air blower for the tip of a dental intraoral scanner, particularly designed to remove saliva, bubbles, blood or fluids that could interfere with the image obtained. The innovative compressed air blower consists of compressed air conduction cannulas with an air inlet connected to the conduction hose or triple syringe or other compressed air source and which develop into two air outlets that surround the scanner tip from the side, simultaneously reaching the object to be scanned such as the tooth and/or gum, simultaneously reaching the object to be scanned, such as teeth and/or gums, thus enabling the air jets conducted by the cannula outlets to eliminate any saliva, bubbles, blood or other fluids, making it possible to obtain a two-dimensional or three-dimensional image free of interferences that could cause distortions in the scanned images.

### HISTORY OF THE INVENTION

It is known that intraoral scanning allows the reproduction of the oral region, especially the dental arch and tissues, for visualization in two-dimensional or three-dimensional form obtained by a scanner camera that films and digitizes the images. This intraoral scanning technique can be carried out before the patient begins treatment, where there is a need for records of their mouth for prior planning, and is widely used for making prostheses, crowns, implants, bruxism mouthguards, customized appliances and others.

Conventional scanners comprise a hand-held tool made up of a base body that serves as an elongated handle, at one end of which the scanning unit or tip is attached, responsible for reading the dental arch or part of it and transforming it into data that is sent to a computer equipped with a data processor, transforming it into very high quality two-dimensional or three-dimensional images displayed on a monitor. The scanning unit or tip that is part of the optical assembly is responsible for capturing the images that will be converted into the software.

Each scanner model can have a different type of tip, which can be removable, fixed or disposable. In general, these include mirrors on the extreme inside, which must always be clean, dry, well attached and free of any damage, such as scratches or stains, so as not to directly affect the quality of the image capture.

Despite the precautions taken, especially with regard to the mirror, the accuracy of intraoral image capture through data collection for the conversion of two-dimensional/three-dimensional images can be affected, since the tip is generally used in a humid region, i.e., inside the patient's mouth, and thus with the constant presence of saliva, bubbles, blood or other fluids, causing blurring during data scanning and, consequently, inaccurate or distorted results.

One current way of eliminating bubbles is to blast them with air using conventional air hoses from the equipment of the professional. However, this procedure is ineffective, as it does not prevent new bubbles from forming during the scan.

### ANALYSIS OF THE STATE OF THE ART

A search of specialized databases found documents related to dental intraoral scanners, such as document no. BR 11.2019.026283-0 which deals with a window system for an intraoral scanner comprising an optical element with a thermal conductivity of more than 1 W m-1 K-1. A window, comprising a panel made of, for example, plastic, glass or corundum, is detachably arranged in the optical element at an average distance of less than 1 mm. At least one heat source is also connected to the optical element. The invention also relates to an intraoral scanner. The intraoral scanner comprises the window system. The optical element and at least one heat source are connected to the intraoral scanner. The window is arranged in a cover. Said cover can be arranged on the intraoral scanner so that the window has an average distance of less than 1 mm from the optical element.

Document no. US2020405140A1 discloses an intraoral scanner comprising a device body and a connection part. The body of the device has a heat dissipation structure and a first electrical connection port. The connection part is connected in a detachable manner to the body of the device and includes a second electrical connection port and a fan. The second electrical connection port is electrically connected to the first electrical connection port. The fan generates a stream of air that flows through the heat dissipation structure.

The aforementioned document, although it shows a fan for producing air, is designed to dissipate heat so that the surface temperature can be reduced to below the temperature required by safety standards.

Document no. TW201817363A presents a defogging device suitable for an intraoral scanner. The intraoral scanner includes a body portion and a terminal portion. The terminal portion is provided with a light inlet surface and a reflective surface. The reflective surface is inclined in relation to the light inlet surface. The defogging device includes a seat body and an energy source. The energy source is arranged in the seat body, adjacent to the reflective surface and supplies energy to the reflective surface to heat the reflective surface. The defogging device comprises a body and an energy generator.

Said document no. TW201817363A presents a defogging device adjacent to the reflective surface, i.e., a mirror supplying energy to the reflective surface for defogging, as distinct from the currently innovated blower whose main function is to remove saliva flow, blood and other fluids.

Therefore, it is a fact that the documents cited in the paragraphs above, despite belonging to the same field of application, do not present any of the characteristics of the object being applied for, thus ensuring that it meets the legal requirements for patentability.

### OBJECTIVES OF THE INVENTION

It is, therefore, the objective of the present invention to present a compressed air blower for the tip of a dental intraoral scanner comprised of at least one pair of air conduction cannulas whose inlet is connected to the conduction hose coupled to the triple syringe or other source of compressed air; these cannulas having free and angled ends, for air outlets aligned with the opening of the scanner tip and the object to be scanned, such as teeth/gums, making it possible. Thus, the air jets conducted through the exits of the cannulas eliminate any accumulation of saliva, bubbles, blood or other fluids, making it possible to obtain a two-dimensional or three-dimensional image free from interference that can cause distortions in the images.

Another objective of this invention is to present a compressed air blower for a dental intraoral scanner tip, whose innovative and simplified characteristics make it possible to produce, assemble and market it at a reduced cost in order to guarantee a wide range of purchases.

It is also the objective of this invention to present a compressed air blower for a dental intraoral scanner tip that can be applied or not to said tip, leaving it up to the professional, when they deem it necessary.

Therefore, the objective of the invention is to remove any saliva or expelled blood that may be present in the oral region in order to reduce interference in data collection when scanning the oral region, thus enabling the generation of perfect three-dimensional and/or two-dimensional images in order to be more effective in aiding dental treatment.

### DESCRIPTION OF THE FIGURES

To complement the present description in order to obtain a better understanding of the characteristics of the present invention and according to a preferential practical realization of the same, a set of drawings is attached to the description, where, in an exemplary but not limiting way, the following has been represented:

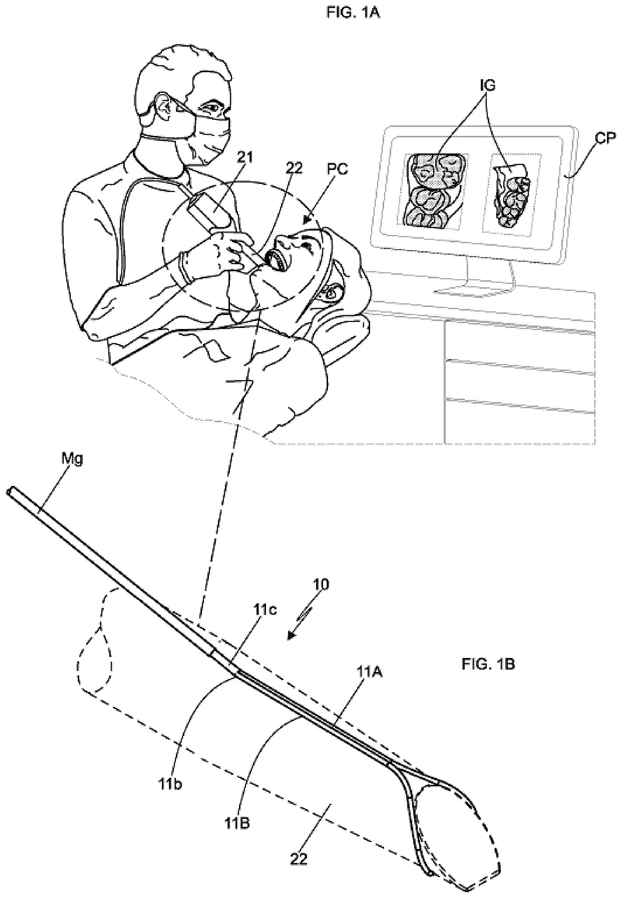

Figure 1A shows a schematic view illustrating the use of the scanner on the patient with the integrated blower;
Figure 1B shows the top view of an enlarged detail of the scanner tip, illustrating the integration of the required compressed air blower;
Figure 1C shows the bottom view of an enlarged detail of the scanner tip, illustrating the integration of the compressed air blower;
Figure 2A shows the bottom view of the blower in question;
Figure 2B shows a top view of the blower;
Figure 2C shows a side view of the blower integrated into the scanner tip;
Figure 2D illustrates a longitudinal cross-sectional view A.A revealing the blower and air conduction to the opening of the scanner tip;
Figure 3A shows a top view of a constructive variation of the blower with three air outlets;
Figure 3B shows a bottom view of the constructive variation of the blower with three air outlets;
Figure 3C shows a top view of the variation of the blower integrated into the scanner tip;
Figure 3D shows a longitudinal section B.B indicated in figure 3C;
Figure 3E shows a cross-section C.C, also shown in figure 3C; and
Figure 4 represents a version of the blower assembly so that it can be injected together with the scanner tip body.

### DESCRIPTION OF THE INVENTION

The present patent of invention relates to the "COMPRESSED AIR BLOWER FOR INTRAORAL DENTAL SCANNER TIP" (20), of the type used to obtain three-dimensional/ two-dimensional images (IG) of the oral region of the patient (PC). This intraoral scanner (20) consists of a pistol (21) and a tip (22), both connected to a computer (CP) by cable or other suitable means.

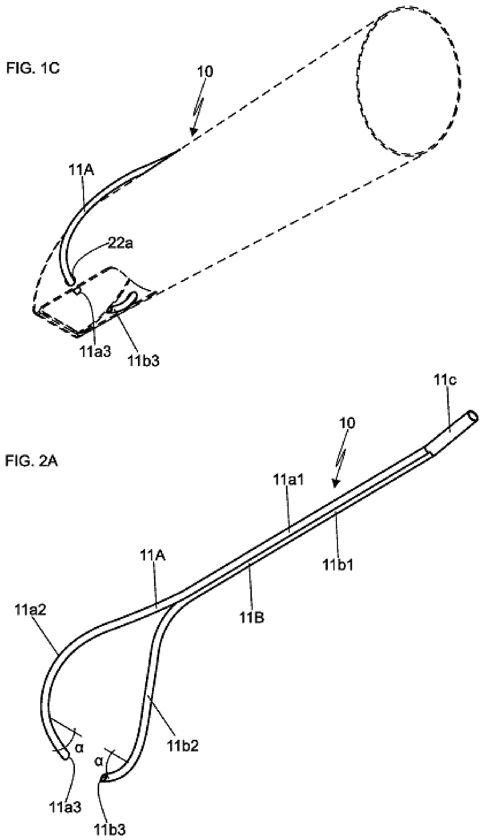

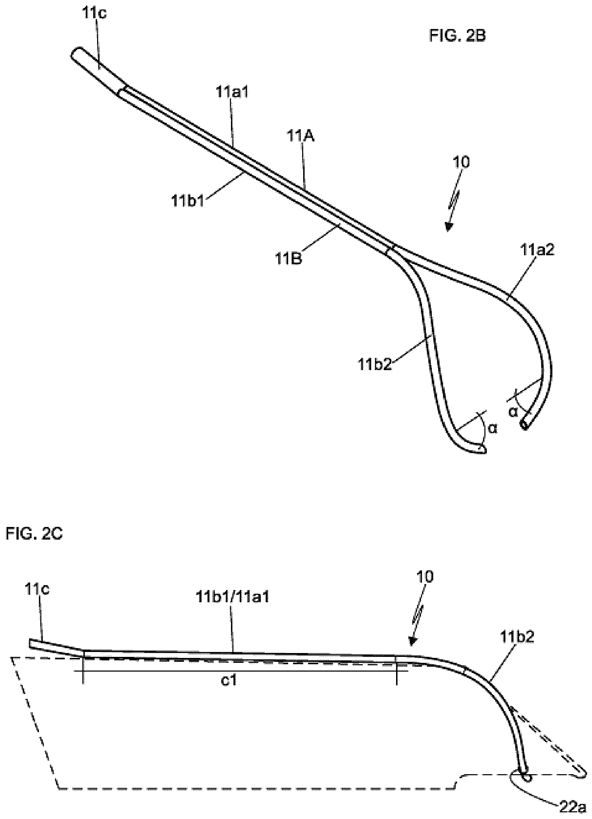

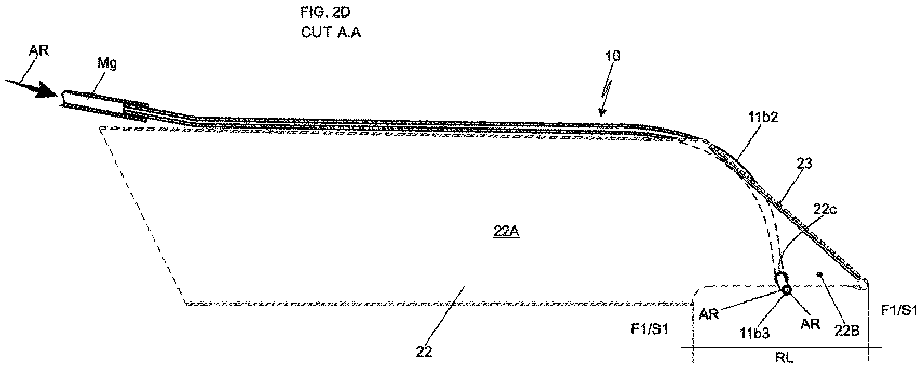

According to the present invention, the compressed air blower (10) is coupled to the body of the tip (22) of the dental scanner (20) and comprises at least a pair of cannulas (11A) and (11B) arranged parallel to each other and axial along the tip (22) with the inlet ends (11a)/(11b) integrated into a nozzle (11c) for coupling to the compressed air (AR) hose (Mg) coupled to the triple syringe (not shown) or other compressed air (AR) source. These cannulas (11A) and (11B) extend in a unified manner in straight sections (11a1) and (11b1) (Fig. 2A), and separate into individual sectors (11a2) and (11b2) which are slightly arched so as to symmetrically surround the end of the tip (22). The free ends of the sections (11a2) and (11b2) are bent obliquely at an angle (α) of 30 to 50 degrees, one facing the other, and have hollow holes (11a3) and (11b3) providing a means for compressed air (AR) to escape. The rectilinear sections (11a1) and (11b1) can vary in length (c1) according to the tip model (22) of the dental scanner (20).

Each compressed air (AR) outlet hole (11a3) and (11b3) is arranged in corresponding openings (22a) located in the lower portion of the side walls (22A) of the tip body (22) and aligned with the opening (22B) where the mirror (23) and cameras (not shown) of the scanner (20) are installed and which are in contact with the moist area of the patient's mouth (PC) when scanning.

The air jets (AR) coming out of the openings (11a3) and (11b3) of the cannulas (11A) and (11B) make up a region (RL) around the opening (22B) of the tip (20) devoid of salivary flow (F1), blood (S1) or any other fluid, a region (RL) of approximately 1 cm² for collecting data from the aforementioned region (RL) and its surroundings free of interference, which is transformed into distortion-free (IG) three-dimensional/ two-dimensional images of the patient's mouth region (PC).

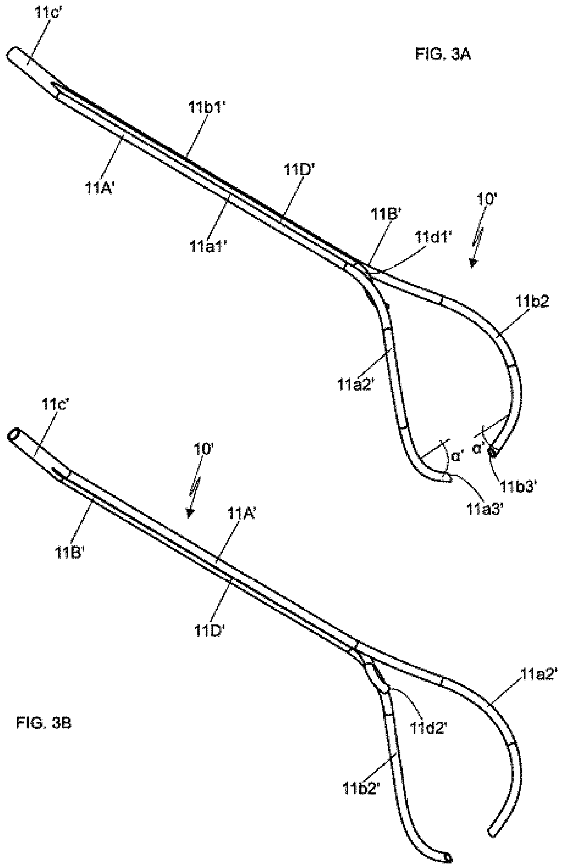

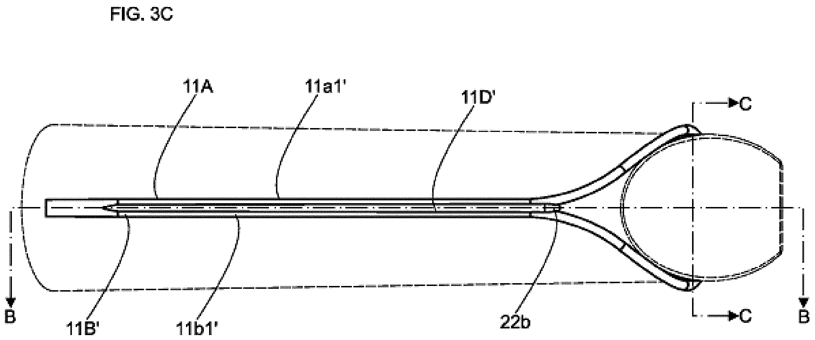

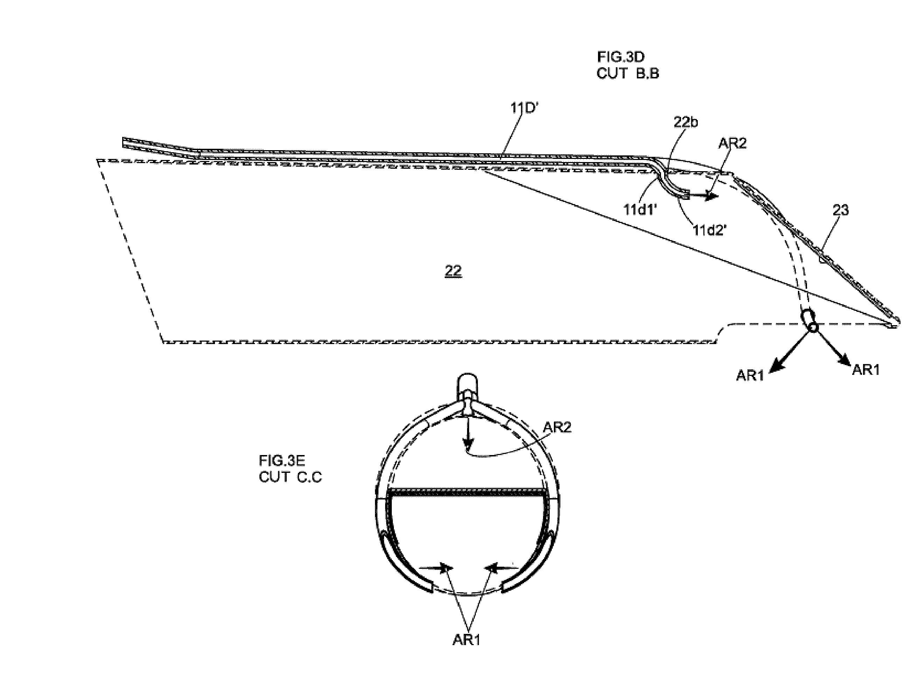

In a second variation (see figures 3A to 3E), the blower (10') is made up of three cannulas (11A'), (11B') and (11D') arranged parallel to each other and which are integrated into a nozzle (11c') that receives the hose (Mg), with these (11A') and (11B') extending in rectilinear stretches (11a1') and (11b1'), which separate into individual slightly arched sections (11a2') and (11b2') with oblique ends and angles (α') of 30 to 50 degrees and openings (11a3') and (11b3') arranged in the openings (22a) practiced in the lower portion of the side walls (22A) of the tip body (22), while the central cannula (11D') is shorter and has an oblique end (11d1') installed in a hole (22b) in the upper portion of the tip (22).

The opening (11d2') of the central cannula (11D') is aligned frontally with the mirror (23) of the scanner (20), making up three air outlets: two side outlets (AR1) for removing saliva (F1), blood (S1) or any other fluid away from the opening (22B) and an air outlet (AR2) for defogging the mirror (23).

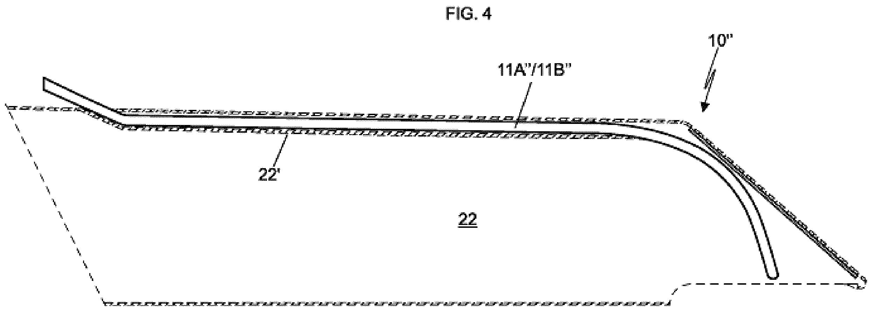

In a third variation (see figure 4), the cannulas (11A") and (11B") that make up the blower (10") are embedded in the wall (22') of the body that makes up the scanner (22).

It is true that when the present invention is put into practice, modifications may be made to certain details of construction and shape, without this implying a departure from the fundamental principles that are clearly substantiated in the claim table, thus understanding that the terminology used was not intended as a limitation.

## Claims

1. "COMPRESSED AIR BLOWER FOR DENTAL INTRAORAL SCANNER TIP" for obtaining three-dimensional/two-dimensional images (IG) of the patient's oral region (PC); said intraoral scanner (20) comprises a pistol portion (21) and tip (22), both connected to a computer (CP) by cable or other suitable means; **characterized by** a compressed air blower (10) formed by a pair of canicles (11A) and (11B) coupled to the body of the tip (22) of the dental scanner (20) composing a region (RL) around the opening (22B) devoid of salivary flow (F1), blood (S1) or any other fluid, region (RL) for data collection and obtaining undistorted (IG) three-dimensional/two-dimensional images of the patient's oral region (PC) using air jets (AR) coming from openings (11a3) and (11b3) of the cannulas (11A) and (11B).

2. "COMPRESSED AIR BLOWER FOR DENTAL INTRAORAL SCANNER TIP", according to claim 1, **characterized by** region (RL) being approximately 1 cm².

3. "COMPRESSED AIR BLOWER FOR DENTAL INTRAORAL SCANNER TIP", according to claim 1, **characterized in that** the pair of canicles (11A) and (11B) are arranged parallel to each other and axially along the tip (22) and which have the inlet ends (11a)/(11b) integrated into a nozzle (11c) coupling the hose (Mg) carrying compressed air (AR) coupled to the triple syringe or other source of compressed air (AR); said canicles (11A) and (11B) extend in a unified manner in rectilinear sections (11a1) and (11b1) and separate into individual sectors (11a2) and (11a2) slightly arched so as to symmetrically surround the end of the tip (22); the free ends of the sectors (11a2) and (11b2) are bent obliquely at an angle (α) of 30 to 50 degrees, facing each other, and have hollow holes (11a3) and (11b3) providing a means for compressed air (AR) to escape; each hole (11a3) and (11b3) for the compressed air (AR) outlet is arranged in corresponding openings (22a) practiced in the lower portion of the side walls (22A) of the tip body (22) and aligned with the opening (22B).

4. "COMPRESSED AIR BLOWER FOR DENTAL INTRAORAL SCANNER TIP", according to claim 3, **characterized by** rectilinear sections (11a1) and (11b1) being able to vary in length (c1) according to the tip model (22 ) of the dental scanner (20).

5. "COMPRESSED AIR BLOWER FOR DENTAL INTRAORAL SCANNER TIP", according to claims 1 and 3 and in a second variation, **characterized by** a blower (10') made up of three cannulas (11A'), (11B') and (11D') arranged in parallel and which are integrated into a nozzle (11c') receiving a hose (Mg), said nozzles (11A') and (11B') developing in a extended manner in rectilinear sections (11a1') and (11b1'), which separate into individual, slightly arched sections (11a2') and (11b2') with oblique ends angled (α') from 30 to 50 degrees and openings (11a3') and (11b3') arranged in the openings (22a) practiced in the lower portion of the side walls (22A) of the body of the tip (22), while the central canicle (11D') is shorter and has an oblique end (11d1') installed in an opening (22b) provided in the upper portion of the tip (22); the opening (11d2') of the central cannula (11D') is aligned frontally with the mirror (23) of the scanner (20), making up three air outlets, two of which are side outlets (AR1) for removing saliva (F1), blood (S1) or any other fluid away from the opening (22B) and an air outlet (AR2) for defogging the mirror (23)

6. "COMPRESSED AIR BLOWER FOR DENTAL INTRAORAL SCANNER TIP", according to claims 1 and 3 and in a third variation, **characterized by** a blower (10") providing cannulas (11A") and (11B") embedded in the wall (22') of the body making up the scanner (22).
